# EUROPÄISCHE PATENTSCHRIFT

(11) **EP 1 210 602 B1**
(45) Veröffentlichungstag und Bekanntmachung des Hinweises auf die Patenterteilung: **01.09.2004**
(21) Anmeldenummer: 00964022.8
(22) Anmeldetag: 17.08.2000
(51) Int. Cl.: G01N 33/564, G01N 33/68

(54) **VERFAHREN ZUR DIAGNOSE VON SJÖGREN-SYNDROM**
METHOD OF DIAGNOSING SJÖGREN'S SYNDROME
PROCEDE DE DIAGNOSTIC DU SYNDROME DE SJOGREN

(30) Priorität: 20.08.1999 DE 19939575
(43) Veröffentlichungstag der Anmeldung: 05.06.2002
(73) Patentinhaber: Orgentec Diagnostika GmbH, 55129 Mainz (DE); Witte, Torsten, 30629 Hannover (DE)
(72) Erfinder: MATTHIAS, Torsten, 55237 Flonheim (DE); WITTE, Torsten, 30629 Hannover (DE)
(74) Vertreter: Weiss, Wolfgang, Dipl.-Chem. Dr.
(86) Internationale Anmeldenummer: PCT/EP2000/008051
(87) Internationale Veröffentlichungsnummer: WO 2001/014877

(56) Entgegenhaltungen:
- QUAN C P ET AL: "Natural polyreactive secretory immunoglobulin A autoantibodies as a possible barrier to infection in humans." INFECTION AND IMMUNITY, (1997 OCT) 65 (10) 3997-4004. , XP000982212
- ISHIMARU NAOZUMI ET AL: "Estrogen deficiency accelerates autoimmune exocrinopathy in murine Sjogren's syndrome through Fas-mediated apoptosis." AMERICAN JOURNAL OF PATHOLOGY, Bd. 155, Nr. 1, Juli 1999 (1999-07), Seiten 173-181, XP000986725 ISSN: 0002-9440
- MIYAGAWA SACHIKO ET AL: "Neonatal lupus erythematosus: Maternal IgG antibodies bind to a recombinant NH2-terminal fusion protein encoded by human alpha-fodrin cDNA." JOURNAL OF INVESTIGATIVE DERMATOLOGY, Bd. 111, Nr. 6, Dezember 1998 (1998-12), Seiten 1189-1192, XP000986884 ISSN: 0022-202X in der Anmeldung erwähnt
- WATANABE TAKAHIRO ET AL: "Anti-alpha-fodrin antibodies in Sjogren syndrome and lupus erythematosus." ARCHIVES OF DERMATOLOGY, Bd. 135, Nr. 5, Mai 1999 (1999-05), Seiten 535-539, XP000986733 ISSN: 0003-987X in der Anmeldung erwähnt
- WITTE TORSTEN ET AL: "IgA and IgG autoantibodies against alpha-fodrin as markers for Sjogren's syndrome." JOURNAL OF RHEUMATOLOGY, Bd. 27, Nr. 11, November 2000 (2000-11), Seiten 2617-2620, XP000986732 ISSN: 0315-162X

## Beschreibung

Die Erfindung betrifft ein Verfahren zur Diagnose von Sjögren-Syndrom und einen dafür geeigneten Reagenzienkit.

Das Sjögren-Syndrom ist eine chronische Systemerkrankung, charakterisiert durch Trockenheit der Augen (Keratoconjunctivits sicca), des Mundes (Xerostonia sicca) und anderer Schleimhäute. Wenn das Sjögren-Syndrom nur die Augen und den Mund erfaßt, spricht man von einem primären Sjögren-Syndrom. Beim sekundären Sjögren-Syndrom treten zusätzlich als Komplikation verschiedene Erkrankungen des rheumatischen Formenkreises, z.B. rheumatoide Athritis, Sklerodermie und Lupus erythematodes auf. Die Prävalenz der Erkrankung schwankt zwischen ca. 0,1 bis 0,5 % der Bevölkerung.

Die Diagnostik des Sjögren-Syndroms erfolgt nach bestimmten Klassifikationskriterien, die
(1) okuläre Symptome,
(2) orale Symptome,
(3) okuläre Befunde, d.h. positive Schirmer- oder Rose-Begal-Test,
(4) histologische Befunde,
(5) Befunde an der Speicheldrüse, beispielsweise aufgrund von Speicheldrüsenbiopsie und
(6) Nachweis von Autoantkörpern, z.B. Anti-Ro/SSA oder Anti-La/SSB, antinukleäre Antikörper oder Rheumafaktoren, umfassen.

In den USA müssen alle der genannten sechs Kriterien für eine Diagnose des Sjögren-Syndroms erfüllt sein, während in Europa nur vier dieser sechs Kriterien genügen.

Da Autoantikörper eine große Rolle beim Sjögren-Syndrom spielen, ist man seit Jahren bemüht einen spezifischen Marker für diese Erkrankung zu finden. Die unter Punkt 6 oben aufgeführten Autoantikörper sind jedoch unspezifisch und kommen auch bei vielen anderen Erkrankungen des rheumatischen Formenkreises vor.

Autoantikörper gegen α-Fodrin wurden in Patienten mit Sjögren-Syndrom nachgewiesen (Haneji et al. Science 276 (1997), 604-607; Miyagawa et al., J. Invest. Dermatol. 111 (1998) 1189-1192 und Watanabe et al., Dermatol. 135 (1999), 535-539). α-Fodrin-Autoantikörper der Immunglobulinklasse G wurden in Patienten mit Sjögren-Syndrom, aber auch in Patienten mit Lupus erythematodes mittels Immunblot gefunden. Diese Befunde weisen daraufhin, daß IgG-Antikörper gegen α-Fodrin keine spezifische Assoziation mit dem Vorhandensein der Sjögren-Syndroms haben. Außerdem wurden die Daten an einer relativ geringen Anzahl an Patienten erhoben. In einer Publikation wurde ein Patientenkollektiv aus 43 Patienten mit primärem und 8 Patienten mit sekundärem Sjögren-Syndrom getestet. In allen nachfolgenden Publikationen wurden 9 Patienten mit primärem Sjögren-Syndrom und 15 Patienten mit sekundärem Sjögren-Syndrom getestet.

Bei eigenen Untersuchungen der Erfinder an größeren Patientenkollektiven (94 Patienten mit Sjögren-Syndrom, 352 Patienten mit systemischem Lupus erythematodes und 160 Kontrollpersonen) konnte die hohe Spezifität von IgG-Autoantikörpern gegen α-Fodrin für das Sjögren-Syndrom nicht bestätigt werden. Statt dessen konnte gezeigt werden, daß IgA-Autoantikörper gegen α-Fodrin eine wesentlich höhere Spezifität aufweisen, nämlich 99,7% für das primäre Sjögren-Syndrom.

Die Erfindung betrifft somit ein Verfahren zur Diagnose von Sjögren-Syndrom, wobei man in einer Probe, die üblicherweise aus einem zu untersuchenden Patienten stammt, das Vorhandensein oder/und die Menge von IgA-Autoantikörper gegen α-Fodrin bestimmt.

Das Auftreten signifikanter Mengen von IgA-Autoantikörpern gegen α-Fodrin ist hochspezifisch für das Vorhandensein von Sjögren-Syndrom, insbesondere von primärem Sjögren-Syndrom, d.h. eine falsch positive Diagnose kann weitgehend ausgeschlossen werden. Das Verfahren hat im übrigen auch eine hohe Sensitivität von etwa 70%, die noch verbessert werden kann, wenn - zusätzlich zur IgA-Bestimmung - das Vorhandensein oder/und die Menge von Autoantikörpern anderer Immunglobulinklassen, z.B. IgG oder/und IgM, gegen α-Fodrin bestimmt wird. So kann beispielsweise durch eine zusätzliche Bestimmung von IgG-Autoantikörpern die Sensitivität des Verfahrens noch um 10% erhöht werden.

Das erfindungsgemäße Verfahren kann als qualitative oder quantitative Bestimmung durchgeführt werden. Bei einer qualitativen Bestimmung werden IgA-Autoantikörper-Konzentrationen, die oberhalb einem sogenannten Cut-Off-Wert liegen, als positiv klassifiziert. Die Bestimmung des Cut-Off-Werts kann durch Kalibrierung des Testsystems mit positiven und negativen Kontrollproben erfolgen. Alternativ kann auch eine quantitative Bestimmung durchgeführt werden.

Die zu testende Probe ist im allgemeinen eine humane Körperflüssigkeit, von der bekannt ist, daß sie IgA-Antikörper und ggf. andere Antikörper enthalten kann. Beispiele für geeignete Körperflüssigkeiten sind Blut, Serum, Plasma oder Speichel, wobei Serum besonders bevorzugt ist.

Die Bestimmung der IgA-Antikörper gegen α-Fodrin kann nach beliebigen auf dem Sachgebiet bekannten Testformaten erfolgen. Vorzugsweise verwendet man ein α-Fodrin-Antigen und einen IgA-spezifischen Rezeptor. Das α-Fodrin-Antigen kann ein natives Protein, welches aus humanen Zellinien erhältlich ist, oder ein rekombinantes Antigen sein, welches in einer heterologen Wirtszelle, z.B. einer Bakterienzelle wie E.coli oder einer eukaryontischen Wirtszelle wie einer Insektenzelle durch rekombinante Proteinexpression hergestellt wurde. Vorzugsweise verwendet man ein rekombinantes α-Fodrin Antigen, welches die Sequenz des nativen α-Fodrins oder Teile davon, insbesondere den N-terminalen Abschnitt enthält. Das rekombinante Antigen kann darüber hinaus heterologe Peptid- oder Polypeptiddomänen enthalten, z.B. eine poly-His-Sequenz, welche die Aufreinigung nach der Expression erleichtert.

Der IgA-spezifische Rezeptor ist im allgemeinen ein Antikörper, der in der Lage ist, Immunglobuline der Klasse A in Gegenwart von Immunglobulinen anderer Klassen, z.B. G oder/und M, selektiv zu erkennen. Für diesen Zweck können polyklonale Anti-IgA-Antiseren verwendet werden, die durch Immunisierung von Versuchstieren, z. B. Ziegen, Ratten, Mäusen, Kaninchen etc. mit humanen IgA nach bekannten Methoden erhältlich sind. Ebenso können jedoch entsprechende monoklonale Anti-IgA-Antikörper eingesetzt werden.

Wie bereits ausgeführt, ist das spezifische Testformat im allgemeinen unkritisch. Vorzugsweise wird jedoch ein heterogenes Testformat verwendet, besonders bevorzugt ein heterogenes Testformat, bei dem ein Immunkomplex bestehend aus α-Fodrin-Antigen, nachzuweisendem IgA-Autoantikörper und IgA-spezifischem Rezeptor an eine Festphase gebunden wird (Sandwich-Testformat). Ebenso kann jedoch auch ein kompetitives Testformat gewählt werden.

Bei einem heterogenen Sandwich-Testformat kann man
(a) ein auf der Festphase immobilisiertes α-Fodrin-Antigen und einen markierten IgA-spezifischen Rezeptor oder
(b) einen auf der Festphase immobilisierten IgA-spezifischen Rezeptor und ein markiertes α-Fodrin-Antigen verwenden.

Als Festphasen können Reaktionsgefäße, Mikrotiterplatten, Beads, Biochips etc. eingesetzt werden. Die Immobilisierung des Antigens bzw. des Rezeptors auf der Festphase kann durch adsorptive Wechselwirkungen, kovalente Bindung oder vermittelt über ein hochaffines Bindepaar (Streptavidin/Biotin, Hapten/Anti-Hapten-Antikörper) erfolgen. Das immobilisierte Testreagenz kann in einer bereits festphasengebundenen Form eingesetzt oder aber auch erst im Verlauf des Tests immobilisiert werden.

Das Verfahren kann als Flüssigtest (z.B. in einem Reaktionsgefäß) oder auch als Trockentest (z.B. auf einem Teststreifen) durchgeführt werden.

Das markierte Testreagenz kann selbst eine nachweisbare bzw. signalgebenden Gruppe tragen (direkte Markierung) oder mit einer nachweisbaren Gruppe bindefähig sein (indirekte Markierung). Die Markierungsgruppe kann beliebig aus allen aus dem Stand der Technik für immunologische Nachweisverfahren bekannten Markierungsgruppen ausgewählt werden, beispielsweise aus Enzymen, Metall- oder Latexpartikeln, sowie lumineszierenden oder fluoreszierenden Gruppen. Besonders bevorzugt wird die Markierungsgruppe aus Enzymen, z.B. Peroxidase, β-Galactosidase oder Alkalische Phosphate ausgewählt und das Verfahren im ELISA-Format durchgeführt.

Noch ein weiterer Gegenstand der Erfindung ist ein Testkit zur Diagnose von Sjögren-Syndrom umfassend
(a) ein α-Fodrin-Antigen und
(b) einen IgA-spezifischen Rezeptor.

Weiterhin kann der Testkit (c) eine Festphase umfassen, an die eines der Testreagenzien (a) oder (b) gebunden ist oder bindefähig ist. Darüber hinaus umfaßt der Testkit vorzugsweise (d) eine Markierungsgruppe, die an eines der Testreagenzien (a) oder (b) gebunden ist oder damit bindefähig ist.

Außerdem kann der Testkit (e) mindestens ein weiteres Antikörperklassen-spezifisches Testreagenz enthalten, falls neben IgA-Autoantikörpern auch noch α-Fodrin-Autoantikörper anderer Immunglobulinklassen bestimmt werden sollen. Beispiele für solche Antikörperklassen-spezifische Testreagenzien sind Anti-IgG-Antikörper oder Protein G zur selektiven Bindung von IgG-Autoantikörpern bzw. Anti-IgM-Antikörper zur selektiven Bindung von IgM-Autoantikörpern. Der Testkit kann darüber hinaus noch weitere übliche Reagenzien wie Puffer, Substrate und Waschlösungen enthalten.

Weiterhin soll die Erfindung durch die nachfolgenden Beispiele verdeutlicht werden:

### Beispiele

### 1. Material und Methoden

### 1.1 Seren

Seren von Patienten mit primärem Sjögren-Syndrom wurden in Austin, Texas (n = 49), Freiburg, Deutschland (n = 20) und Hannover, Deutschland (n = 18) gesammelt. Die Diagnose von Sjögren-Syndrom war in Austin nach den US-amerikanischen San Diego Kriterien und in Deutschland nach den modifizierten europäischen Kriterien für die Klassifizierung von Sjögren-Syndrom erfolgt.

Seren von Patienten mit Systemischen Lupus erythematodes (SLE) mit oder ohne sekundärem Sjögren-Syndrom wurden in Hannover gewonnen. Die Diagnose von sekundärem Sjögren-Syndrom erfolgte unter Verwendung der modifizierten europäischen Klassifikationskriterien.

Zusätzlich wurden Aliquots von 352 tiefgefrorenen Serumproben verwendet, die zuvor für eine deutsche SLE-Untersuchung gesammelt worden waren. Alle 352 Patienten erfüllten mindestens vier der ACR-Kriterien für die Diagnose von SLE (Tan et al., Arthr. and Rheum. 25 (1982), 1271-1277). Überlappende Syndrome wurden ausgeschlossen. In vorhergehenden Untersuchungen waren die Patienten ausführlich hinsichtlich ihrer Klinik- und Laborparameter charakterisiert worden. Da einige dieser Parameter nicht bei allen 352 Patienten bestimmt worden waren, erfolgten die Korrelationen mit den verfügbaren Daten, wobei für jeden Klinikparameter Daten von mindestens 339 Patienten und für jeden Laborparameter Daten von mindestens 1 Patienten verwendet wurden.

160 Seren von Blutspendern wurden als Kontrolle für die Spezifität des Nachweisverfahrens untersucht.

### 1.2 Nachweis von Antikörpern gegen α-Fodrin durch einen ELISA

Die cDNA für den N-terminalen Abschnitt von α-Fodrin wurde aus der von einer humanen Speicheldrüse isolierten mRNA durch PCR kloniert. Die Primer hatten die Position 93-130 (upstream) und 1827-1882 (downstream), wobei ein Konstrukt mit 1731 bp erhalten wurde (Nummerierung entsprechend Moon et al., J. Biol. Chem. 265 (1991) 4427-4433). Die cDNA wurde in prokaryontische und eukaryontische Expressionsvektoren (pet32b (Novagen) bzw. pVL1393 (Pharmingen)) kloniert und in Form eines His-Tag-Fusionsproteins in E.coli und Sf9 Insektenzellen exprimiert. Das rekominante Protein wurde zur Beschichtung von ELISA-Platten verwendet.

Die Seren wurden 1:100 in einem Verdünnungspuffer pH 7,4 (75 mM NaCl, 0, 1 % Tween 20) verdünnt. 100 µl der verdünnten Seren wurden 30 min auf den ELISA-Platten inkubiert. Nach 3 Waschschritten mit Verdünnungspuffer unter Verwendung eines automatisierten ELISA Waschgeräts (SLT Labinstruments, Grödig, Österreich) wurde mit Meerrettichperoxidase markiertes Ziegenantiserum, das für IgG oder IgA spezifisch war, für 15 min zugegeben. Nach drei weiteren Waschschritten mit Verdünnungspuffer wurden 100 µl Tetramethylbenzidin als Substrat für einen Zeitraum von 15 min zugegeben. Die Reaktion wurde durch Zugabe von 100 µl 1 M HCl gestoppt und die Extinktion (OD) bei 450 nm unter Verwendung eines ELISA Auswertungsgeräts (Rainbow Reader SLT-Labinstruments, Grödig, Österreich) bestimmt.

Um einen Standard für den ELISA zu bestimmen, wurden zehn Seren von Patienten mit primären Sjögren-Syndrom vermessen. Die Konzentration von α-Fodrin-Antikörpern in dem Serum mit der höchsten OD wurde willkürlich mit 100 U/ml definiert. Dieses Serum wurde als Laborstandard verwendet.

Zum täglichen Kalibrierung wurde dieses Referenzserum in Konzentrationen entsprechend 0, 12, 5, 25, 50 und 100 U/ml verwendet. Um eine Standardkurve zu erhalten, wurden die gemessenen OD-Werte in einer logarithmisch/linearen Skala aufgetragen.

### 1.3 Nachweis von Rheumafaktoren sowie der Autoantigene Ro und La

IgG-, IgA- und IgM-Rheumatoidfaktoren sowie Autoantikörper gegen Antigene Ro und La wurden unter Verwendung kommerziell erhältlicher ELISA Systeme gemäß den Vorschriften des Herstellers (ORGenTec GmbH, Mainz, Deutschland) bestimmt.

### 1.4 Statistische Auswertung

Das Vorhandensein von IgG- und IgA-Antikörpern gegen α-Fodrin in den 352 von SLE Patienten erhaltenen Seren wurde mit Klinik- und Laborparametern in Korrelation gebracht.

Für die statistische Auswertung wurden nicht-parametrische Tests verwendet, da die Verteilung von Rheumafaktoren eindeutig von einer Gausschen Verteilung abwich. Die Korrelation von Antikörpern gegen α-Fodrin mit Klinik- und Laborparametern wurde unter Verwendung des Chi-Quadrat-Tests bestimmt. Eine Assoziierungswahrscheinlichkeitvon weniger als 0,05 wurde als statistisch signifikant angesehen.

### 2. Ergebnisse

### 2.1 Verteilung von IgA und IgG Antikörpern gegen α-Fodrin in Patienten und Kontrollproben

In 160 Seren von Blutdonoren war der Mittelwert ( ± Standardabweichung) der Konzentration von IgA- bzw. IgG-Antikörpern gegen α-Fodrin 9,2 U/ml ± 5,5 U/ml bzw. 11,1 U/ml ± 7,2 U/ml. Der Prozentsatz von Seren mit Antikörpern gegen α-Fodrin wurde unter Verwendung eines Cut-Off-Werts berechnet, der als mittlere Konzentration von Antikörpern gegen α-Fodrin in den Seren von Blutdonoren plus 3 Standardabweichungen definiert wurde (entsprechend 25 U/ml für IgA-Antikörper und 32 U/ml für IgG-Antikörper).

Mittels dem in 1.2 beschriebenen ELISA-Test wurden IgA-Antikörper gegen α-Fodrin in 53 der 83 Seren von Patienten von primärem Sjögren-Syndrom identifiziert (64%). In 7 von 15 Seren aus Patienten mit SLE und Sjögren-Syndrom wurden ebenfalls IgA-Antikörper gegen α-Fodrin gefunden (47%).

IgA-Antikörper gegen α-Fodrin waren nur in einem von 160 Blutdonor-Seren und in einem von 50 Seren von SLE Patienten ohne Sjögren-Syndrom nachweisbar. Daraus ergab sich eine Testspezifität von 99,7%.

IgG-Antikörper gegen α-Fodrin wurden in 48 von 83 Seren aus Patienten mit primären Sjögren-Syndrom nachgewiesen (57%). In 6 von 15 Seren aus Patienten mit SLE und sekundärem Sjögren-Syndrom konnten ebenfalls IgG-Antikörper gegen α-Fodrin gefunden werden (40%).

IgG-Antikörper gegen α-Fodrin wurden auch in 3 von 160 Blutdonor-Seren und in keinem von 50 Seren aus SLE Patienten ohne Sjögren-Syndrom nachgewiesen.

10% der Patienten mit primärem Sjögren-Syndrom hatten nur IgG-Antikörper, aber keine IgA-Antikörper gegen α-Fodrin.

Es konnte keine Korrelation des Vorhandenseins von IgG- und IgG-Anti-α-Fodrin-Antikörpern mit Autoantikörpern gegen Ro, La, und IgA- bzw. IgG-Rheumafaktoren in den 83 Patienten mit primärem Sjögren-Syndrom gefunden werden.

### 2.2 Assoziation von IgA- und IgG-Antikörpern gegen α-Fodrin mit SLE-Klinikparametern

Das Vorhandensein von IgA-Antikörpern gegen α-Fodrin zeigte eine positive Korrelation mit den Parametern Erythem (p < 0,01) und Fötalverlust (p<0,05). Das Vorhandensein von IgG-Antikörpern gegen α-Fodrin korrelierte mit kutaner Vasculitis (p < 0,05) und Arthralgie (p < 0,05).

### 2.3 Assoziation von IgG- und IgA-Antikörpern gegen α-Fodrin mit SLE-Laborparametern

Das Vorhandensein von IgA-Antikörpern gegen α-Fodrin zeigte eine positive Korrelation mit den Parametern erhöhte IgA-Konzentration (p<0,001), erhöhte IgM Konzentration (p < 0,05), Neutropenie (p < 0,05), IgG-Antikörper gegen Cardiolipin (p<0,01), IgA (p<0,001) und IgM (p<0,05) Autoantikörper gegen β2 Glykoprotein und IgG Rheumafaktoren (p < 0,05). IgG Antikörper gegen α-Fodrin korrelierten nur schwach mit einer positiven Critidie-Reaktion (p<0,05).

## Patentansprüche

1. Verfahren zur Diagnose von Sjögren-Syndrom,
**dadurch gekennzeichnet,**
**daß** man in einer aus einem Patienten stammenden Probe das Vorhandensein oder/und die Menge von IgA-Autoantikörpern gegen α-Fodrin bestimmt.

2. Verfahren nach Anspruch 1,
**dadurch gekennzeichnet,**
**daß** man als Probe eine humane Körperflüsskeit, insbesondere Blut, Serum, Plasma oder Speichel, verwendet.

3. Verfahren nach Anspruch 1 oder 2,
**dadurch gekennzeichnet**,
daßman zur Bestimmung der IgA-Autoantikörper ein α-Fodrin-Antigen und einen IgA-spezifischen Rezeptor verwendet.

4. Verfahren nach einem der Ansprüche 1 bis 3,
**dadurch gekennzeichnet,**
**daß** man ein heterogenes Testformat verwendet.

5. Verfahren nach Anspruch 4,
**dadurch gekennzeichnet,**
**daß** man ein Sandwich-Testformat verwendet.

6. Verfahren nach einem der Ansprüche 4 bis 5,
**dadurch gekennzeichnet,**
**daß** man
(a) ein immobilisiertes α-Fodrin-Antigen und einen markierten IgA-spezifischen Rezeptor oder
(b) einen immobilisierten IgA-spezifischen Rezeptor und ein markiertes α-Fodrin-Antigen verwendet.

7. Verfahren nach Anspruch 6,
**dadurch gekennzeichnet,**
**daß** die Markierungsgruppe ausgewählt wird aus Enzymen, Metalloder Latexpartikeln sowie lumineszierende oder fluoreszierende Gruppen.

8. Verfahren nach einem der vorhergehenden Ansprüche,
**dadurch gekennzeichnet,**
**daß** man weiterhin das Vorhandensein oder/und die Menge von Autoantikörpern anderer Immunglobulinklassen gegen α-Fodrin bestimmt.

9. Verfahren nach Anspruch 8.
**dadurch gekennzeichnet,**
**daß** man weiterhin IgG- oder/und IgM-Autoantikörper gegen α-Fodrin bestimmt.

10. Testkit zur Diagnose von Sjögren-Syndrom umfassend
(a) ein α-Fodrin-Antigen und
(b) einen IgA-spezifischen Rezeptor.

11. Testkit nach Anspruch 10, weiterhin umfassend
(a) eine Festphase, an die eine der Testragenzien (a) oder (b) gebunden oder bindefähig ist.

12. Testkit nach Anspruch 10 oder 11, weiterhin umfassens
(d) eine Markierungsgruppe, die an eines der Testreagenzien (a) oder (b) gebunden oder damit bindefähig ist.

13. Testkit nach einem der Ansprüche 10 bis 12 weiterhin umfassend
(e) mindestens ein weiteres Antikörperklassen-spezifisches Testreagenz.

## Claims

1. Method for diagnosing Sjögren's syndrome,
**characterized in that**
the presence or/and the amount of IgA autoantibodies against α-fodrin is determined in a sample derived from a patient.

2. Method as claimed in claim 1,
**characterized in that**
a human body fluid and in particular blood, serum, plasma or saliva is used as the sample.

3. Method as claimed in claim 1 or 2,
**characterized in that**
an α-fodrin antigen and an IgA-specific receptor are used to determine the IgA autoantibodies.

4. Method as claimed in one of the claims 1 to 3,
**characterized in that**
a heterogeneous test format is used.

5. Method as claimed in claim 4,
**characterized in that**
a sandwich test format is used.

6. Method as claimed in one of the claims 4 to 5,
**characterized in that**
(a) an immobilized α-fodrin antigen and a labelled IgA-specific receptor or
(b) an immobilized IgA-specific receptor and a labelled α-fodrin antigen are used.

7. Method as claimed in claim 6,
**characterized in that**
the labelling group is selected from enzymes, metal or latex particles and luminescent or fluorescent groups.

8. Method as claimed in one of the previous claims,
**characterized in that**
the presence or/and the amount of autoantibodies of other immunoglobulin classes against α-fodrin is additionally determined.

9. Method as claimed in claim 8,
**characterized in that**
IgG or/and IgM autoantibodies against α-fodrin are additionally determined.

10. Test kit for diagnosing Sjögren's syndrome comprising
(a) an α-fodrin antigen and
(b) an IgA-specific receptor.

11. Test kit as claimed in claim 10, additionally comprising
(a) a solid phase to which one of the test reagents (a) or (b) is bound or can be bound.

12. Test kit as claimed in claim 10 or 11, additionally comprising
(d) a labelling group which is bound or can be bound to one of the test reagents (a) or (b).

13. Test kit as claimed in one of the claims 10 to 12 additionally comprising,
(e) at least one other antibody class-specific test reagent.

## Revendications

1. Procédé de diagnostic du syndrome de Sjögren **caractérisé en ce que** l'on détermine la présence et/ou la quantité d'auto-anticorps IgA contre l'α-fodrine dans un échantillon provenant d'un patient.

2. Procédé selon la revendication 1 **caractérisé en ce que** l'on utilise comme échantillon un liquide biologique humain, en particulier le sang, le sérum, le plasma ou la salive.

3. Procédé selon la revendication 1 ou 2 **caractérisé en ce que** l'on utilise un antigène d'α-fodrine et un récepteur spécifique d'IgA pour la détermination des auto-anticorps IgA.

4. Procédé selon l'une des revendications 1 à 3 **caractérisé en ce que** l'on utilise un format de test hétérogène.

5. Procédé selon la revendication 4 **caractérisé en ce que** l'on utilise un format de test sandwich.

6. Procédé selon l'une des revendications 4 à 5 **caractérisé en ce que** l'on utilise
(a) un antigène d'α-fodrine immobilisé et un récepteur spécifique d'IgA marqué ou
(b) un récepteur spécifique d'IgA immobilisé et un antigène d'α-fodrine marqué.

7. Procédé selon la revendication 6 **caractérisé en ce que** le groupe de marquage est choisi parmi les enzymes, les particules métalliques ou de latex ainsi que les groupes luminescents ou fluorescents.

8. Procédé selon l'une des revendications précédentes **caractérisé en ce que** l'on détermine en outre la présence et/ou la quantité d'auto-anticorps d'autres classes d'immunoglobulines contre l'α-fodrine.

9. Procédé selon la revendication 8 **caractérisé en ce que** l'on détermine en outre des auto-anticorps IgG et/ou IgM contre l'α-fodrine.

10. Trousse de test pour le diagnostic du syndrome de Sjögren comprenant
(a) un antigène d'α-fodrine et
(b) un récepteur spécifique d'IgA.

11. Trousse de test selon la revendication 10 comprenant en outre
(a) une phase solide à laquelle l'un des réactifs de test (a) et (b) est lié ou peut se lier.

12. Trousse de test selon la revendication 10 ou 11 comprenant en outre
(d) un groupe de marquage qui est lié ou qui peut se lier à l'un des réactifs de test (a) et (b).

13. Trousse de test selon l'une des revendications 10 à 12 comprenant en outre
(e) au moins un autre réactif de test spécifique de classes d'anticorps.
